(19) Europäisches Patentamt
European Patent Office
Office européen des brevets

(11) Publication number: 0 254 000 B1

(12) **EUROPEAN PATENT SPECIFICATION**

(45) Date of publication of patent specification: **22.05.91**

(51) Int. Cl.⁵: **C12Q 1/34**, C12Q 1/04, //G01N33/571

(21) Application number: **87108148.5**

(22) Date of filing: **05.06.87**

(54) **Rapid detection of N. gonorrhoeae without culture.**

(30) Priority: **16.06.86 US 874499**

(43) Date of publication of application: **27.01.88 Bulletin 88/04**

(45) Publication of the grant of the patent: **22.05.91 Bulletin 91/21**

(84) Designated Contracting States: **DE FR GB**

(56) References cited:
**EP-A- 122 023**
**US-A- 4 018 653**
**US-A- 4 166 765**
**US-A- 4 242 447**
**US-A- 4 390 622**

(73) Proprietor: **MILES INC.**
**1127 Myrtle Street**
**Elkhart Indiana 46514(US)**

(72) Inventor: **Gantzer, Mary Lou**
**25723 Kiser Court**
**Elkhart Indiana 46514(US)**

(74) Representative: **Dänner, Klaus, Dr. et al**
**c/o Bayer AG Konzernverwaltung RP Patentabteilung**
**W-5090 Leverkusen 1 Bayerwerk(DE)**

**EP 0 254 000 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

I. Field of the Invention

The present invention relates generally to diagnostic methods and compositions for the determination of the presence of *N. gonorrhoeae*. In particular, it relates to the rapid detection of *N. gonorrhoeae* in a body fluid sample.

II. Utility

The containment of gonorrhea is of worldwide concern. Since 1975, the annually reported number of cases in the U.S. alone has been approximately one million. However, the actual incidence is estimated to be between 1.6 and 2.0 million.

There are several reasons why present test methods for the detection of gonorrhea are inadequate. First, unless gonorrhea symptoms appear, testing under current regimens is unlikely. Therefore, asymptomatic patients, in particular females, are seldom diagnosed early. Approximately 15% of infected women exhibit no symptoms until the disease is quite advanced and even then the symptomatology can be mistaken for other problems which require dramatically different treatment.

Second, most methods currently used involve a delay in diagnosis because of the methods themselves or because of the sample used. The consequences of delayed diagnosis are severe for the population since the disease is highly infectious and can be very severe for the patient if untreated.

Two test methods are widely used currently: gram stain and culture of a specimen of urethral exudate or cervical secretion followed by culture confirmation methods. Gram staining identifies the presence of intracellular gram negative diplococci which in conjunction with certain symptomatology in males is considered definitive enough to begin treatment for gonorrhea. If gram staining does not produce evidence of intracellular gram negative diplococci in a symptomatic male, culture of urethral exudate on specialized media designed to allow growth of the genus *Neisseria*, of which *gonorrhoeae* is one species, and confirmation of the presence of *N. gonorrhoeae* in the colonies by an oxidase test or other accepted culture confirmation procedures is used. A test which identifies high oxidase content is sometimes used directly on urethral exudate of the symptomatic male as a presumptive test for gonorrhea. Culture of cervical secretion and subsequent confirmation of any colony is the method of choice for females because the incidence of false negatives is very high with a gram stain of cervical secretion and too many of the normal female flora exhibit the oxidase positive characteristic for that test to provide even a presumptive indication of gonorrhea. Culturing requires approximately 48 hours before confirmation techniques can be used.

When the possibility of a gonorrhea infection is determined, the case must be reported to the State Health Department which in turn reports the case to the Centers for Disease Control. In addition, a local agency interviews the infected person to determine who else might have been infected. Each suspected contact is traced. Because of the requirement for a sample of urethral exudate or cervical secretion for testing, the contact cannot be tested where found but must be asked to cooperate and report to a clinic or physician.

In addition to the delay from the time a contact is traced and the contact reports and is tested, test results for females are not available for two days. If positive, the contact must be asked to return for treatment and a case history to identify her possible contacts.

In contrast, the test of this invention is so simple that trained personnel are not necessary either to collect the sample or to perform and interpret the results; in addition the test results can be available in less than an hour. These advantages make the test method suitable as a tool for screening asymptomatic patients, in particular females, and as a tool for immediate onsite testing for persons who have been named as sexual contacts of a gonorrhea positive patient.

III. Information Disclosure

Presumptive identification of *N. gonorrhoeae* is made by culturing a specimen on selective media. Colonies having the appearance of *N. gonorrhoeae* are then tested for oxidase activity and gram stained. Due to problems inherent in culturing, false negative results are obtained in as many as 50 to 70% of specimens cultured.

Gram stain alone is often used on the symptomatic males. However, the possibility of false negatives is

high for females or asymptomatic males.

The oxidase positive test has also been advanced as a screening test without prior culture. Since Chlamydia, the second most likely cause of similar symptomatology in males, is not oxidase positive, an oxidase test is effective for symptomatic males; for the female, the number of oxidase positive organisms found in the cervical secretions is too great to permit direct diagnostic use of the oxidase test.

U.S. Patent 4,208,480 to D'Amato et al. shows a method and device for the differential detection of *N. gonorrhoeae* from an isolate of a specimen which has been subjected to culture on a selective medium. This isolate from a single large colony of suspected *N. gonorrhoeae* is then added to a test composition including a β-naphthylester, such as β-naphthyl valerate or a β-naphthylamide, and an indicator system. The isolate and composition mixture are then incubated again at about 35° C for up to 5 hours, at which point a positive or negative reaction is determined by comparison with an identification chart. D'Amato et al. essentially disclose a culture confirmation method.

Another rapid confirmation method for N. gonorrhoeae using glycyl-propyl-naphtylamide is disclosed in the EP-A-122023 to Chu, Albert En-Yuen.

U.S. Patent No. 3,876,503 and 4,108,653 to Mennen describes a nonculture method and instrument for a rapid presumptive test for *N. gonorrhoeae*. The test is performed on male urethral exudate. The exudate on a swab is contacted with a dry, porous, absorbent pledget impregnated with a phenylenediamine. The pledget which has been contacted with sample is inserted into a tube of transparent plastic having flexible side walls which contains a frangible ampule containing a physiological salt solution. The ampule is broken so that the salt solution wets the pledget. A color change obtained 30 to 120 seconds after wetting indicates a positive presumptive test for gonorrhea. U.S. Patent No. 4,108,729 to Mennen discloses a paper booklet composed of staggered strips, one strip impregnated with reagents, preferably N,N,N',N'-tetramethyl-p-phenylenediamine. The strip is said to provide a presumptive test for gonorrhea when contacted with male urethral exudate. Both tests are based on a positive response to the presence of oxidase in the sample.

## IV. Summary of the Invention

The present invention provides a rapid diagnostic method for detecting the presence of *N. gonorrhoeae* in a urine sample or other body fluid, comprising the steps of:

a) contacting the urine sample with a test composition comprising,

(i) an enzyme substrate capable of being cleaved by the enzymatic activity of *N. gonorrhoeae* to produce a cleavage product;

(ii) a buffer capable of providing a pH of from about 6.5 to 8.5 when contacted with the sample; and

b) determining the cleavage product by production of a detectable response.

This method provides a test for a urine sample which can be collected without an examination room or trained personnel. The method can be used to detect the presence of *N. gonorrhoeae* in symptomatic or clinically asymptomatic patients, including females. Several test compositions are also provided which produce the preferred optical response, i.e., color formation.

## V. Detailed Description of Invention

Currently disclosed methods for the determination of the presence of *N. gonorrhoeae* require the use of either urethral exudate or a single colony isolate obtained from culture of a body fluid sample on primary isolate media. Culturing, although very time consuming and labor intensive, was believed necessary to separate suspected *N. gonorrhoeae* colonies from other bacteria and to increase the number of bacteria prior to performing confirmation procedures.

It has now been found that the use of urethral exudate and/or culture of a body fluid sample is not required to detect gonorrhea infection even in females and clinically asymptomatic males. It has been found that a urine sample contains enough *N. gonorrhoeae* bacteria to provide positive results with the method of this invention. Oral-pharyngeal fluids, cervical secretions and urethral exudate can also be tested with this method. This method is not limited to symptomatic male patients as are those methods requiring the use of male exudate. The ability to detect the presence of a gonorrhea infection with a urine specimen makes this method useful as a screening method.

Generally, the method of this invention is based on the use of a urine sample, with results available in less than about one hour. The urine sample is contacted with a test composition comprising a buffer capable of providing a suitable pH for enzyme activity when contacted with the sample and a substrate

which can be cleaved by enzymatic activity of *N. gonorrhoeae*. Cleavage of the provided substrate produces a cleavage product which is detectable directly or indirectly with the addition of components which react with the cleavage product to produce a detectable response. Because of the ease of detection, the production of color is preferred. The color formed can then be determined either visually or instrumentally by reflectance or absorbance, depending on the test format.

Buffers are commonly used to assure the proper pH for enzymatic activity within the test composition. They are particularly important for urine sample testing since such samples exhibit widely varying pH and are often too acidic for good enzymatic activity. The preferred pH range is from pH 6.5 to 8.5. The buffer incorporated should be capable of providing such a pH in the test composition when contacted with the sample. Suitable buffers include citrate, phosphate and N-tris(hydroxymethyl)methyl-2-anilino-ethane sulfonic acid (TES). However, optimization of the buffer component is within the skill of the art given the present disclosure.

A. Direct Color

One enzyme associated with *N. gonorrhoeae* is proline iminopeptidase which can cleave di- and tripeptides containing proline at the amino terminal end. By supplying the substrate L-prolyl-*p*-nitroanilide or its equivalent containing a longer peptide chain, *p*-nitroaniline, a yellow compound, is produced as a cleavage product in the presence of *N. gonorrhoeae*. The production of this cleavage product can be detected visually or instrumentally.

B. Indirect Color

Many cleavable substrates can be designed so that a detectable response can be obtained by the interaction of the cleavage product with a coupling component.

1. Carboxylic Acid Ester Substrate

Carboxylic acid esters, having the following structure, can be cleaved by enzymatic activity associated with *N. gonorrhoeae*.

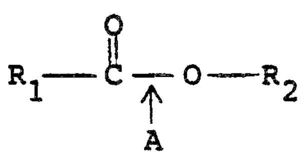

The substrate is cleaved at A to form a cleavage product, $R_2$, which can react with a coupling component to produce color. Useful compounds include those wherein $R_1$ is an aliphatic carbon chain of from about 1 to 10 carbon atoms and $R_2$ is β-naphthyl, pyrrole, phenyl or a thiophene moiety.

Carboxylic acid esters wherein $R_2$ is a pyrrole or thiophene moiety are preferred, thiophene being particularly preferred. β-naphthyl esters such as β-naphthyl caproate, β-naphthyl caprylate, β-naphthyl propionate, β-naphthyl oleate and β-naphthyl valerate can also be used. Of the β-naphthyl-carboxylic acid esters, β-naphthyl caproic acid ester is preferred. Substituted β-naphthyl moieties can also be used as the $R_2$ group.

Table 1 shows which coupling components are useful with specified $R_2$ groups.

TABLE 1

| $R_2$ | Coupling Component |
|---|---|
| β-naphthyl | diazonium salt |
| | 4-aminoantipyrene/ ferricyanide |
| phenyl | diazonium salt |
| | 2,5-dichloroquinone-4-chlorimide |
| pyrrole | diazonium salt |
| thiophene | diazonium salt |

The ferricyanide used with 4-aminoantipyrene can be various alkali metal ferricyanides such as sodium or potassium ferricyanide. The above indicator systems all produce the preferred optical response, color, when contacted with a specimen containing *N. gonorrhoeae*.

Suitable diazonium salts are listed in Table 2 below with their common names or abbreviations. However, this list should not be taken as limiting as many diazonium salts are available.

Table 2

| | |
|---|---|
| MMBD | 2-methoxy-4-morpholino benzenediazonium chloride |
| fast blue RR | 4-benzoylamino-2,5-dimethoxyaniline |
| fast blue B | o-dianisidine, tetrazotized |
| fast red B | 2-methoxy-4-nitro-benzenediazonium chloride |
| fast red TR | 5-chloro-2-toluidine diazonium chloride |
| fast red RC | diazo-5-chloro-o-anisidine |
| fast blue BB | 4'-amino-2',5'-diethoxybenzanilide |

Preferably an activator is also included in the test composition with the carboxylic acid ester and the

diazonium salt. Useful activators include alcohols such as isopropanol, methanol, hexanol, propanol, 1,8-octanediol, decanol and ethanol. Alcohols are commonly used in concentrations of 4 to 2 M. Isopropanol is a preferred activator in a test composition containing 9-naphthyl caproic acid ester and a diazonium salt. The concentration of the diazo compound can range from 0.10 to 2 mg/mL, preferably 0.2 to 0.5 mg/mL.

A preferred test composition containing β-naphthyl caproic acid ester, 4-benzoylamino-2,5-dimethoxyaniline and isopropanol has been used in a solution format to determine the presence of *N. gonorrhoeae* in clinical urine samples. Results were available in less than 10 minutes as compared with 2 days with previous methods when culture of the sample was presumed necessary. The color formed can be compared to a sample blank and the color difference assessed visually or spectrophotometrically by absorbance at 518 nanometers. In this study, a difference of 0.085 absorbance units between the sample blank (substrate omitted) and the test solution was indicative of a positive sample. This composition did not respond to the presence of leukocytes or E. coli, possible urine contaminants known to exhibit esterase activity.

2. β-naphthylamides

β-naphthylamides of L-proline or L-hydroxyproline can be cleaved by the proline iminopeptidase or hydroxyproline aminopeptidase activity of *N. gonorrhoeae.* Color is formed when a diazonium salt such as those listed previously is added to the test composition to produce color.

3. Proline thiobenzyl ester

Proline thiobenzyl esters can be cleaved by the proline iminopeptidase activity of *N. gonorrhoeae* When 5,5'-dithiobis-2-nitrobenzoic acid is added to the test composition as a coupling component, a yellow compound, 3-carboxy-4-nitrothiophenoxide, can be detected.

C. Coupling Component Plus Enzyme Component

Another detection scheme utilizes an additionally provided enzyme component with the coupling component and buffer to produce a detectable response. The test composition then comprises: (a) substrate which can be cleaved by the enzymatic activity of *N. gonorrhoeae*; (b) a buffer ; (c) a coupling component capable of reacting with the cleavage product; and (d) an enzyme component. An activator, such as those described earlier in the specification, is preferably included. The final result is preferably color.

Examples of this type of reaction are shown below:

I.

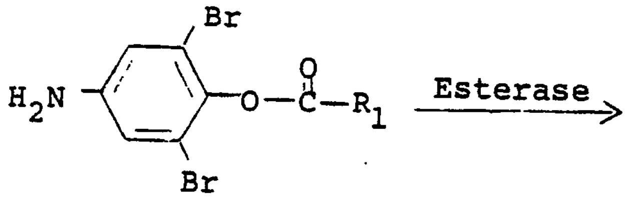


[cleavage product]

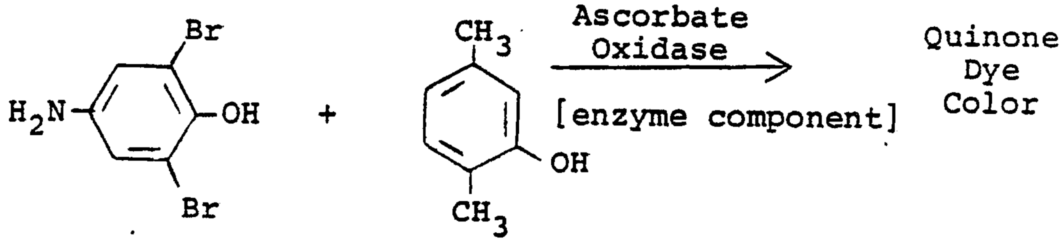


[coupling component]

II.

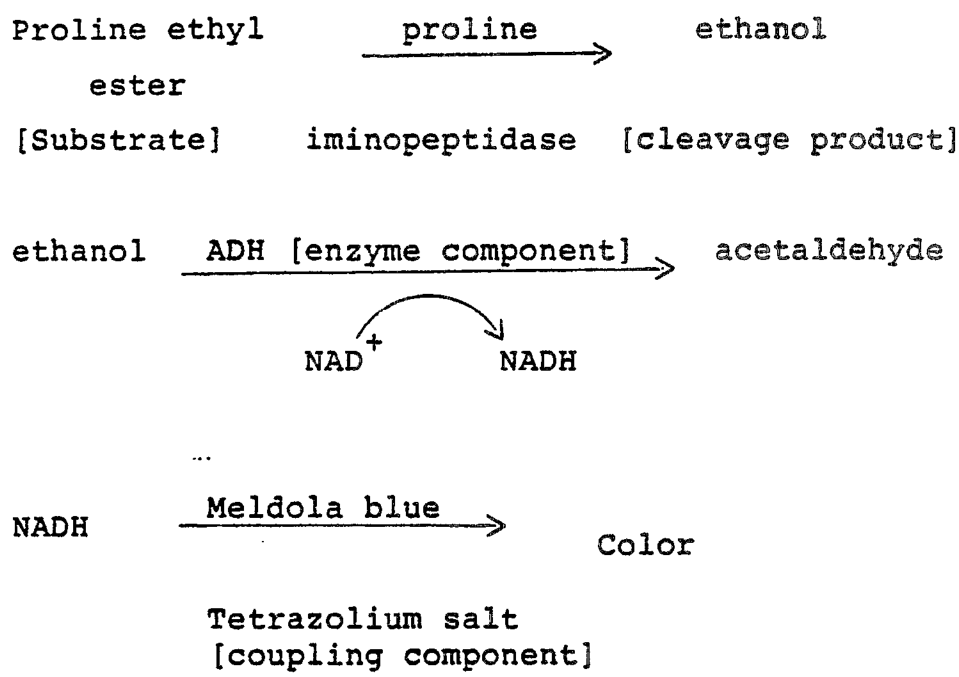


In detection scheme II, reduced nicotinamide adenine dinucleotide [NADH] can be measured spectrophotometrically at 340 nanometers or a coupling component such as meldola blue and a tetrazolium salt can be added to produce color. There are many tetrazolium salts available and their use is known to those skilled in the art.

C. Test Composition

The test composition containing the enzyme substrate, a buffer and, optionally, a coupling component, an activator and an enzyme component can be used by adding it to a urine sample to form a solution. In addition to the test components enumerated previously, other components well known to those skilled in the art such as stabilizers, or preservatives can be added. The reagents can be provided in a "kit" format, e.g., a series of bottles containing one or more of the above components, either in solution, or as a premixed powder, or as a lyophilized powder which can be reconstituted for use as a single working solution.

The composition can also be incorporated into a dry form such as a pressed or molded tablet or a dry reagent test strip. Pressed or molded tablets usually contain filler or bulking agents in addition to the test composition and the tablet is usually easily dissolvable to form a reagent solution. Dry reagent test strips are well known in the art.

To form a dry reagent test strip, a carrier matrix is incorporated with the test composition. Optionally, other components such as wetting agents and thickeners can be added. Wetting agents can facilitate uniform spreading of the specimen over the surface of the carrier. Thickeners are sometimes added to incorporating solutions to increase the amount of solution absorbed by the carrier. The carrier matrix can comprise any substance capable of being incorporated with the components of the test composition, as long as it is substantially inert with respect to the test composition, porous and/or absorbent relative to the aqueous sample to be tested. The expression "carrier matrix" refers to either bibulous or nonbibulous matrices, including film formats, which are insoluble in and maintain their structural integrity when exposed to water or to physiological fluids.

A test strip is often prepared by impregnating a bibulous matrix, e.g., filter paper, with the composition, followed by drying and affixing of the dried impregnated matrix to a support member. The drying can be accomplished by any means which will not deleteriously affect the impregnated composition, usually by means of an air oven. The dried paper is then cut and mounted on one end of a support member, for example, a rigid or semirigid polystyrene film strip. Mounting of the paper on the strip can be accomplished through use of a double-faced adhesive tape, such as those commercially available from the 3M Co., St.

Paul, MN.

A reagent strip test device is advantageously used by momentarily dipping it in a test sample or by otherwise introducing a test sample onto the carrier matrix, whereby a detectable change (e.g., color) results when *N. gonorrhoeae* organisms are present. Depending on the test composition and conditions such as temperature, the results can be available in less than about ten minutes, preferably less than five minutes. Room temperature testing was used routinely since it was considered to be the most useful condition to provide a simple to use screening test. However, a higher temperature could be used provided the necessary equipment was available.

Other formats are also useful. For example part of the reagent composition can be incorporated into a carrier matrix or into a tablet and part can be placed into a test tube. The test tube would usually contain at least the substrate component. The sample would then be added to the test tube to form a solution, incubated for less than about one hour and the tablet or strip dropped or dipped into the solution.

## ABBREVIATIONS

The following abbreviations have been used in the specification for convenience.

| | |
|---|---|
| MMBD | 2-methoxy-4-morpholino benzenediazonium chloride |
| ADH | alcohol dehydrogenase |
| fast blue RR | 4-benzoylamino-2,5-dimethoxyaniline |
| fast blue B | o-dianisidine, tetrazotized |
| fast red B | 2-methoxy-4-nitrobenzene diazonium chloride |
| fast red TR | 5-chloro-2-toluidine diazonium chloride |
| fast red RC | diazo-5-chloro-o-anisidine (also known as diazo red RC) |
| fast blue BB | 4'-amino-2',5'-diethoxy-benzanilide |
| fast green FCF | See Merck Index 10th Ed. #3876 for chemical name |
| fast garnet GBC salt | 4'-amino-2,3'-dimethyl-azobenzene |
| fast red ITR | $N^1,N^1$-diethyl-4-methoxy-metanilamide |
| Triton® X-100 | polyethylene glycol-*p*-isooctylphenyl ether |
| DMF | dimethylformamide |

| | |
|---|---|
| $NAD^+$ | ionized form of NADH |
| NADH | reduced nicotinamide adenine dinucleotide |
| TES | N-tris(hydroxymethyl)-methyl-2-anilinoethane-sulfonic acid |
| v/v | volume per volume: defined herein as mL per 100 mL of solution |
| μL | microliter |
| mL | milliliter |
| mg/mL | milligrams per milliliter |
| mM | millimolar |
| M | molar |
| °C | degrees Centigrade |
| R.T. | room temperature: defined herein as 20 to 25°C |
| A518 | absorbance at 518 nanometers |

The invention will now be illustrated, but is not intended to be limited, by the following examples.

## EXAMPLES

### Preparation of Cell Suspension

Gonococcal isolates were obtained from a local public health clinic and recultured on Thayer-Martin Selective Agar (BBL, Cockeysville, MD) to establish stock cultures. The cultures were harvested and maintained at -70° C in 0.5 to 1.0 mL aliquots of 3% Trypticase® soy broth (BBL) containing 15% glycerol. The cell suspension for use in the assay was prepared by thawing an aliquot of the stock culture, spinning it down to form a cell pellet, removing the supernatant fluid, and then resuspending the pellet in one milliliter of 0.15 M sodium phosphate buffer, pH 7.25. The suspension was used to confirm the reactivity of various test compositions with enzymes associated with *N. gonorrhoeae.*

### EXAMPLE 1

The activity of gonococcal esterase with a β-naphthyl ester substrate was tested with the following assay solutions:

| | |
|---|---|
| Triton X-100, 10% v/v | 100 µL |
| β-naphthyl ester, 50 mM in DMF | 75 µL |
| Phosphate buffer, pH 7.25, 0.3M | 500 µL |
| Fast blue RR, 1.2 mg/mL in DMF | 500 µL |
| Distilled water | 1325 µL |

The reaction was initiated by the addition of 20 µL cell suspension. After a thirty minute incubation at room temperature, the absorbance at 518 nanometers was measured against a sample blank with the following results:

| Substrate | $A_{518}$ |
|---|---|
| β-naphthyl valerate | 0.041 |
| β-naphthyl caproate | 0.080 |

When a stock culture is used as the sample, positive absorbance reading is indicative of sufficient enzymatic activity to provide a test for the presence of N. gonorrhoeae. Therefore, both substrates could be used for the test.

EXAMPLE 2

The esterase activity of the gonococcus organism was tested with β-naphthyl ester substrates of varying chain length. The assay solutions contained an alcohol activator and were prepared as follows:

| | |
|---|---|
| Triton® X-100, 10% v/v | 0.100 mL |
| Ethanol | 0.100 mL |
| β-naphthyl ester, 50 mM in DMF | 0.075 mL |
| Sodium phosphate buffer, 0.3 M, pH 7.25 | 0.500 mL |
| Fast blue RR, 1.2 mg/mL in DMF | 0.500 mL |
| Distilled water | 1.255 mL |

Cell suspension (20 µL) was added to each assay tube, and after a thirty minute incubation at room temperature the absorbance at 518 nanometers was read on a Bausch and Lomb Spectronic 400 spectrophotometer. The data obtained is shown below:

| Substrate | $A_{518}$ |
|---|---|
| β-naphthyl propionate | 0.010 |
| β-naphthyl valerate | 0.061 |
| β-naphthyl caproate | 0.113 |
| β-naphthyl caprylate | 0.049 |

Conclusion: The experiment indicated that the length of the acid portion of the ester affects reactivity. The reactivity of the six carbon caproate ester was the best. Addition or deletion of one carbon from a six carbon chain resulted in approximately 40% decrease in activity.

EXAMPLE 3

The utility of various diazonium salts as indicators for the enzymatic cleavage of β-naphthyl carboxylic acid esters was assessed with visual reading and a four minute incubation at room temperature. Solutions were prepared as follows:

| | |
|---|---|
| Triton® X-100, 10% v/v | 0.100 mL |
| Sodium phosphate buffer, 0.3 M, pH 7.25 | 0.500 mL |
| β-naphthyl caproate, 50 mM in DMF | 0.075 mL |
| Diazonium salt, 1.2 mg/mL in DMF | |
| Isopropanol | 0.300 mL |
| Distilled water | 1.325 mL |

Cell suspension (20 μL) was added to each tube, and the tubes incubated four minutes at room temperature. At the end of this incubation time, the tubes were examined visually for a change in color. The results were as follows:

| Salt | Color Change |
|---|---|
| Fast blue RR | + |
| Fast blue B | + |
| Fast green FCF | - |
| Fast red B | + |
| Fast garnet GBC salt CI 37210 | - |
| Fast red TR | + |
| Fast red ITR | - |
| Diazo red RC (fast red RC) | + |
| Fast blue BB | + |
| Fast red 3GL | - |
| 2-methoxy-4-morpholino-benzene diazonium chloride | - |

The assay conditions used included only a four minute incubation. With this incubation time, some diazonium salts which would be expected to couple with β-naphthol as the cleavage product did not show sufficient color for visual reading. Others, however, provided good visual results with this very brief test time.

EXAMPLE 4

The effect of a number of potential nucleophilic activators of the esterase reaction was examined. The assay solutions were prepared as follows:

| | |
|---|---|
| Triton® X-100, 10% v/v | 0.100 mL |
| Sodium phosphate buffer 0.3M, pH 7.25 | 0.500 mL |
| β-naphthyl caproate, 50 mM in DMF | 0.075 mL |
| Activator | variable (see table) |
| Fast blue RR, 1.2 mg/mL in DMF | 0.500 mL |
| Distilled water | to 2.5 mL final volume |

The reaction was initiated by the addition of 20 μL of cell suspension. After sixty minutes incubation at room temperature, the absorbance of the solutions at 518 nanometers was determined on a Bausch and Lomb Spectronic 400 spectrophotometer. The activation of the enzymatic reaction due to the presence of the nucleophile or activator was calculated as follows:

$$\text{Percent Activation} = \frac{\text{absorbance in presence of nucleophile} - \text{absorbance in absence of nucleophile}}{\text{absorbance in absence of nucleophile}} \times 100$$

The results obtained are tabulated below:

| Activator | Concentration | % Activation |
|---|---|---|
| Isopropanol | 1.57 M | 84 |
| Methanol | 3.95 M | 56.8 |
| Quinine Hydrochloride (Sigma) | 4.0 mM | 51.6 |
| Hexanol | 6.36 mM | 43.1 |
| Propanol | 1.6 M | 42.3 |
| Propylene Glycol | 1.63 M | 41 |
| 1,8-Octanediol (Aldrich) | 4.0 mM | 40.4 |
| Decanol | 2.1 mM | 37.2 |
| Ethanol | 1.71 M | 37 |
| 1,10-Phenanthroline (Sigma) | 2.0 mM | 22.1 |
| L-2-Amino-3-phenyl-1-propanol (Aldrich) | 2.0 mM | 19.2 |
| 1,10-Decanediol (Aldrich) | 4.0 mM | 15.4 |
| Quinuclidinol | 0.9 mM | 9 |

The first nine compounds listed were the best alcohol activators when β-naphthyl caproate was used as a substrate. Isopropanol was a preferred activator for solution assays. However, a nonvolatile alcohol, such as decanol, is preferred in a reagent strip format.

EXAMPLE 5

A pyrrole substrate, 3-caproyloxy-5-phenyl-pyrrole having the structure shown below was used in the

method of the invention.

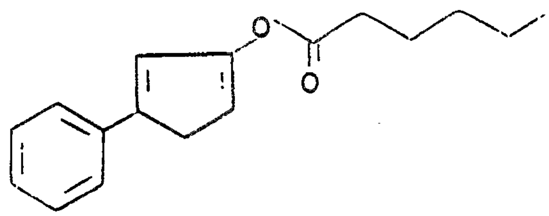

A test composition was prepared:

| | |
|---|---|
| pyrrole ester | 4mM |
| Triton® X-100 | 1% |
| isopropanol | 10% |
| citrate buffer, pH 6.5 | 50 mM |

The composition was placed in a series of assay tubes and 50 μL of gonococcal cell suspension was added to each tube. After one minute incubation at room temperature, 4 mg/dL fast blue RR was added. The resulting mixtures were incubated from one to five minutes at room temperature, and then the absorbance at 550 nm of each was read against a reagent blank. The results were as follows:

| Time | $A_{550}$ |
|---|---|
| 1 min. | 0.040 |
| 5 min. | 0.093 |

The pyrrole substrate provided a detectable response after 5 minutes which can distinguish the presence of *N. gonorrhoeae.*

EXAMPLE 6

A test solution was prepared using a thiophene enzyme substrate:

| | |
|---|---|
| TES buffer, pH 8 | 50 mM |
| Triton® X-100 | 2.5% |
| t-butyl alcohol | 15% |
| MMBD | 0.3 mM |
| 3-caproyloxy-5-phenyl thiophene | 4 mM |
| cell suspension | 50 µL |

The structure of the thiophene enzyme substrate used was:

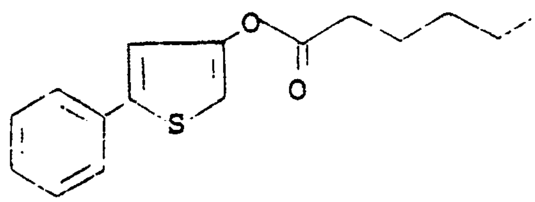

The absorbance was monitored at 545 nanometers. After two minutes at room temperature, the absorbance was 0.321, indicating that this test system effectively detects the presence of *N. gonorrhoeae* without prolonged incubation.

EXAMPLE 7

Clinical confirmation of the method of the invention was obtained with the following experiment. The esterase assay was conducted on fresh urine samples obtained from patients attending a local public health clinic. Control samples (138) were obtained (fresh or frozen) from laboratory personnel, patients attending urology clinic or patients attending a general medical clinic. The clinical samples were treated as follows: samples from females were automatically cultured; exudate samples from symptomatic males were gram stained, and, if the results of gram staining were negative or questionable, the samples were cultured. Control samples were tested only with the esterase assay.

The assay conditions used were:

| | |
|---|---|
| Triton® X-100, 10% v/v | 0.100 mL |
| β-naphthyl caproate, 50 mM in DMF | 0.075 mL |
| Sodium phosphate buffer, 0.3 M, pH 7.25 | 0.500 mL |
| Isopropanol | 0.300 mL |
| Distilled $H_2O$ | 1.025 mL |
| Fast blue RR, 1.2 mg/mL in DMF | 0.500 mL |

Occasionally, unidentified urine components will react with a diazonium salt to produce color. Accordingly, a urine blank was also run on each sample; the conditions were the same as those listed above, with the exception that the β-napthyl caproate was omitted and 0.075 mL DMF used instead.

Reaction was initiated by the addition of 0.05 mL of a urine sample. After a 10 minute incubation at room temperature, the absorbance at 518 nanometers was determined. The assay was considered positive for N. gonorrhoeae if the change in absorbance readings, sample to blank, was greater than or equal to 0.085. The results obtained on patient and control samples are presented in the following table.

Public Health Clinic Urine Samples:

| | | |
|---|---|---|
| 34% | (43) | positive by reference method and esterase assay |
| 43% | (55) | negative by reference method, positive by esterase assay |
| 13% | (17) | negative by reference method, and esterase assay |
| 10% | (13) | positive by reference method negative by esterase assay |

Control Samples

| | | |
|---|---|---|
| 12% | (16) | positive by esterase assay |

The 12% positive samples in the control group is approximately the level of incidence one would anticipate for a low-prevalence population. This low positive level obtained in the control group could indicate that the 43% apparent false positives obtained in the clinic samples indicate failure of the reference method rather than false positive results for the method of the invention.

All urine samples obtained from patients with gonorrhea also contain varying numbers of polymorphonuclear leukocytes (PMN's), which possess a number of esterolytic enzymes, and therefore could present an interference in the assay. A number of urine samples which were judged to be negative for N. gonorrhoeae by culture but which contained PMN's were tested in the assay and yielded negative results, indicating no interference. Low specific gravity samples gave unreliable results, possibly due to low organism content.

Many modifications and variations can be made in the formulations without departing from the spirit or scope of the invention.

## Claims

1. A rapid diagnostic method for detecting the presence of *N. gonorrhoeae* in a urine sample, comprising the steps of:
   (a) contacting the urine sample with a test composition to form a test mixture, the test composition including:
   (i) an enzyme substrate capable of being cleaved by the enzymatic activity of *N. gonorrhoeae* to produce a cleavage product;
   (ii) a buffer capable of providing a pH of from about 6.5 to 8.5 in the test mixture; and (b) detecting the presence of *N. gonorrhoeae* by producing an optical response with the cleavage product.

2. The rapid diagnostic method of claim 1 in which the detectable optical response is color.

3. The rapid diagnostic method of claims 1 and 2 in which the test composition additionally includes a coupling component.

4. The rapid diagnostic method of claim 3 in which the coupling component is a diazonium salt capable of interacting with the cleavage product to produce colorimetric response.

5. The rapid diagnostic method of claims 1-4 in which the enzyme substrate is chosen from the group consisting of $\beta$-naphthyl carboxylic acid esters, pyrrole carboxylic acid esters and thiophene carboxylic acid esters.

6. The rapid diagnostic method of claims 1-5 in which the test composition additionally includes an alcohol activator.

7. The rapid diagnostic method of claims 1-6 in which the test composition additionally includes an enzyme component.

8. A rapid diagnostic method for detecting the presence of *N. gonorrhoeae* in a urine sample, comprising the steps of:
   (a) contacting the urine sample with a test composition to form a test mixture, the test composition including:
   (i) an enzyme substrate capable of being cleaved by the enzymatic activity of *N. gonorrhoeae* to produce a cleavage product;
   (ii) a buffer capable of providing a pH of from about 6.5 to 8.5 in the test mixture; and
   (b) incubating test mixture less than one hour; and
   (c) determining the presence of *N. gonorrhoeae* by producing a colorimetric response with the cleavage product.

9. A test composition for the rapid diagnosis of the presence of *N. gonorrhoeae* in a body fluid sample comprising:
   (a) an enzyme substrate capable of being cleaved by the enzymatic activity of *N. gonorrhoeae* to produce a cleavage product and chosen from the group consisting of thiophene caproate, pyrrole caproate or $\beta$-naphthyl caproate.
   (b) a buffer capable of providing a pH in the range of from about 6.5 to 8.5;
   (c) a diazonium salt capable of coupling with the cleavage product to produce a colorimetric response; and
   (d) an activator.

10. The test composition of claim 9 in which the activator is an alcohol.

## Revendications

1. Méthode de diagnostic rapide pour la détection de la présence de N. gonorrhoeae dans un échantillon d'urine, comprenant les étapes consistant :

(a) à mettre en contact l'échantillon d'urine avec une composition analytique pour former un mélange analytigue, la composition analytique comprenant :

(i) un substrat d'enzyme capable d'être clivé par l'activité enzymatique de N. gonorrhoeae pour engendrer un produit de clivage ;

(ii) un tampon capable d'engendrer un pH d'environ 6,5 à 8,5 dans le mélange analytique ; et

(b) à détecter la présence de N. gonorrhoeae par la production d'une réponse optique avec le produit de clivage.

2. Méthode de diagnostic rapide suivant la revendication 1, dans laquelle la réponse optique détectable est une couleur.

3. Méthode de diagnostic rapide suivant les revendications 1 et 2, dans laquelle la composition analytique comprend en outre un constituant de copulation.

4. Méthode de diagnostic rapide suivant la revendication 3, dans laquelle le constituant de copulation est un sel de diazonium capable d'interagir avec le produit de clivage pour engendrer une réponse colorimétrique.

5. Méthode de diagnostic rapide suivant les revendications 1 à 4, dans laquelle le substrat d'enzyme est choisi dans le groupe comprenant des esters d'acide $\beta$-naphtylcarboxylique, des esters d'acide pyrrolecarboxylique et des esters d'acide thiophène-carboxylique.

6. Méthode de diagnostic rapide suivant les revendications 1 à 5, dans laquelle la composition analytique comprend en outre un activateur du type alcool.

7. Méthode de diagnostic rapide suivant les revendications 1 à 6, dans laquelle la composition analytique comprend en outre un constituant enzymatique.

8. Méthode de diagnostic rapide pour la détection de la présence de N. gonorrhoeae dans un échantillon d'urine, comprenant les étapes consistant :

(a) à mettre en contact l'échantillon d'urine avec une composition analytique pour former un mélange analytique, la composition analytique comprenant :

(i) un substrat d'enzyme capable d'être clivé par l'activité enzymatique de N. gonorrhoeae pour engendrer un produit de clivage;

(ii) un tampon capable d'engendrer un pH d'environ 6,5 à 8,5 dans le mélange analytique; et

(b) à mettre en incubation le mélange analytique pendant un temps inférieur à 1 heure ; et

(c) à déterminer la présence de N. gonorrhoeae par la production d'une réponse colorimétrique avec le produit de clivage.

9. composition analytique pour le diagnostic rapide de la présence de N. gonorrhoeae dans un échantillon d'un liquide biologique, comprenant :

(a) un substrat d'enzyme capable d'être clivé par l'activité enzymatique de N. gonorrhoeae pour engendrer un produit de clivage, et choisi dans le groupe comprenant le caproate de thiophène, le caproate de pyrrole et le caproate de $\beta$-naphtyle;

(b) un tampon capable d'engendrer un pH dans la plage d'environ 6,5 à 8,5;

(c) un sel de diazonium pouvant présenter une copulation avec le produit de clivage pour engendrer une réponse colorimétrique ;

(d) un activateur.

10. Composition analytique suivant la revendication 9, dans laquelle l'activateur est un alcool.

## Ansprüche

1. Schnellnachweisverfahren zur Ermittlung der Gegenwart von N- gonorrhoeae in einer Urinprobe, mit folgenden Schritten:

(a) die Urinprobe wird mit einer Testzusammensetzung zur Bildung einer Testmischung in Kontakt gebracht, wobei die Testzusammensetzung umfasst:

(i) ein Enzymsubstrat, welches durch enzymatische Wirksamkeit von N. gonorrhoeae zur Herstellung eines Spaltungsproduktes gespalten werden kann;
(ii) einen Puffer, der in der Testmischung einen pH-Wert von etwa 6,5 bis 8,5 zur Verfügung stellen kann; und
(b) Ermittlung der Gegenwart von H. gonorrhoeae durch Erzeugung einer optischen Antwort mit dem Spaltungsprodukt.

**2.** Schnellnachweisverfahren nach Anspruch 1, dadurch **gekennzeichnet,** dass die ermittelbare optische Antwort eine Farbe ist.

**3.** Schnellnachweisverfahren nach den Ansprüchen 1 und 2, dadurch **gekennzeichnet,** dass die Testzusammensetzung zusätzlich eine Kupplungskomponente enthält.

**4.** Schnellnachweisverfahren nach Anspruch 3, dadurch **gekennzeichnet,** dass die Kupplungskomponente ein Diazoniumsalz darstellt, welches in der Lage ist, mit dem Spaltungsprodukt unter Erzeugung einer colorimetrischen Antwort in Wechselwirkungen einzutreten.

**5.** Schnellnachweisverfahren nach den Ansprüchen 1 bis 4, dadurch **gekennzeichnet,** dass das Enzymsubstrat aus der Gruppe ausgewählt wird, die aus $\beta$-Naphthylcarbonsäureestern, Pyrrolcarbonsäureestern und Thiophencarbonsäureestern besteht.

**6.** Schnellnachweisverfahren nach den Ansprüchen 1 bis 5, dadurch **gekennzeichnet,** dass die Testzusammensetzung zusätzlich einen Alkoholaktivator enthält.

**7.** Schnellnachweisverfahren nach den Ansprüchen 1 bis 6, dadurch **gekennzeichnet,** dass die Testzusammensetzung zusätzlich eine Enzymkomponente enthält.

**8.** Schnellnachweisverfahren zur Ermittlung der Gegenwart von N. gonorrhoeae in einer Urinprobe, mit folgenden Schritten:
(a) Kontaktieren der Urinprobe mit einer Testzusammensetzung zur Bildung einer Testmischung, wobei die Testzusammensetzung umfasst:
(i) ein Enzymsubstrat, welches durch enzymatische Wirksamkeit von N. gonorrhoeae unter Erzeugung eines Spaltproduktes gespalten werden kann;
(ii) einen Puffer, der in der Lage ist, einen pH-Wert von etwa 6,5 bis 8,5 in der Testmischung zur Verfügung zu stellen; und
(b) Inkubieren der Testmischung für weniger als 1 Stunde; und
(c) Ermittlung der Gegenwart von N. gonorrhoeae durch Erzeugung einer colorimetrischen Antwort mit dem Spaltprodukt.

**9.** Testzusammensetzung für den schnellen Nachweis der Gegenwart von N. gonorrhoeae in einer Körperfluidprobe, umfassend:
(a) ein Enzymsubstrat, welches durch die enzymatische Wirksamkeit von N. gonorrhoeae unter Erzeugung eines Spaltproduktes gespalten werden kann und welches aus der Gruppe ausgewählt ist, die aus Thiophencaproat, Pyrrolcaproat oder $\beta$-Naphthylcaproat besteht;
(b) einen Puffer, der in der Lage ist, einen pH-Wert in dem Bereich von etwa 6,5 bis 8,5 einzustellen;
(c) ein Diazoniumsalz, welches in der Lage ist, mit dem Spaltprodukt zu Kuppeln, unter Erzeugung einer colorimetrischen Antwort; und
(d) einen Aktivator.

**10.** Testzusammensetzung nach Anspruch 9, dadurch **gekennzeichnet,** dass der Aktivator ein Alkohol ist.